# EUROPEAN PATENT APPLICATION

(11) **EP 3 492 910 A1**
(43) Date of publication of application: **05.06.2019**
(21) Application number: 17205203.7
(22) Date of filing: 04.12.2017
(51) Int. Cl.: G01N 21/95, G01N 21/17, G03F 7/20, G01N 29/04, G01N 29/06

(54) **SUBSURFACE INSPECTION METHOD AND SYSTEM**

(71) Applicant: Nederlandse Organisatie voor toegepast- natuurwetenschappelijk onderzoek TNO, 2595 DA 's-Gravenhage (NL)
(72) Inventor: KOEK, Wouter Dick, 2595 DA's-Gravenhage (NL); VAN ZWET, Erwin John, 2595 DA's-Gravenhage (NL)
(74) Representative: V.O.

(57) **Abstract**

A subsurface inspection method and system for detecting internal defects and/or overlay/misalignment in a semiconductor wafer. A measurement laser beam is split into a laser probe beam and a reference laser beam. The laser probe beam is transmitted to a wafer surface. A laser excitation pulse is transmitted impinging the wafer surface for causing an ultrasound wave propagating through the wafer and causing wafer surface movement when reflected back from an encountered subsurface feature. The laser probe beam and the reference laser beam are recombined in an optical interference detector and the subsurface feature inside the wafer is detected by a deviation of a detected phase difference. The laser probe beam and the reference laser beam are guided through an optic prior to arriving at the optical interference detector. The optic has a dispersive characteristic dimensioned to enlarge the phase difference between the reference beam and the wave length shifted probe beam.

## Description

### FIELD OF THE INVENTION

The invention relates to a method and system for subsurface inspection.

### BACKGROUND TO THE INVENTION

A wafer or semiconductor device may require regular inspections for internal (structural) defects, irregularities, or anomalies which may be present inside, for instance to ensure quality, integrity and/or reliability of the wafer. Such subsurface features can be invisible or hidden when for example performing known surface inspection methods. Subsurface inspection or imaging may be required to examine subsurface domains of the wafer for the presence of inhomogeneities or the like, which may strongly influence the functionality, quality and/or the lifetime of the wafer.

Also overlay error between device layers of for example a multilayer semiconductor device may need to be determined. Overlay errors may for example occur as a result of interlayer misalignment between (functional) layers of a multilayer semiconductor device. In the multilayered structure of semiconductor devices, functional device layers are typically deposited on top of other functional device layers. The functional product features, i.e. the features of the pattern formed in each layer, need to be aligned accurately with those in contiguous functional layers to enable correct operation of the end product: the semiconductor device (e.g. wafer). This is achieved by monitoring an overlay error (cf. alignment) during manufacturing by determining relative positions between marker elements in subsequent layers. The term 'overlay error' relates to the amount of misalignment between subsequent layers, and therefore may include offset errors, i.e. errors in the position of a layer in relation to other layers, as well as layer alignment errors, i.e. incorrect orientation of a layer with respect to other layers.

There is a need for a high resolution non-invasive subsurface inspection which allows the detection of small scale structures, such as nanostructures which may be buried inside the wafer, and/or determining an overlay or misalignment error.

In photo-thermal acoustic imaging (PTAI) an excitation laser beam is emitted to a surface of the sample to create an acoustic shock which propagates through the sample. When the acoustic wave is reflected back on a structure inside the sample, the reflected wave can propagate back towards the surface of the wafer and cause a deformation. Information about the three-dimensional (3D) structure of the sample can be inferred by measuring the time and position resolved surface shape of the wafer being deformed as a result of the acoustic wave reflected back from an encountered subsurface feature, such as a defect or anomaly within the sample. The small displacements induced by the reflected wave can be measured by means of an interference-based laser detection system such as a laser interferometer. However, the amplitude of the surface deformation (i.e. deflection) of the wafer is rather small, typically in the order of a few picometers to a few tens of picometers. Such small displacements are approaching or have reached the limits of what is accurately measurable using an interference-based laser detection system such as a laser interferometer. Subsurface features in the sample may be intentionally introduced structures or non-intentional structures (e.g. defect). The intentionally introduced structures of the sample may be determined for identifying overlay/misalignment errors within the sample (e.g. multilayer wafer).

Various efforts have been made to improve the resolution of subsurface imaging. For instance, as a result of decreased signal-to-noise ratios due to the small deflections, many measurements (and subsequent averaging) are performed to obtain more acceptable signal-to-noise ratios. However, the improvement may be limited. It is desirable to further improve the accuracy, signal-to-noise ratio and/or detection limits in PTAI methods and systems. Furthermore, increasing the strength of the (input) acoustic shock wave, such as to increase the amplitudes of the wafer surface deformations resulting from the wave reflected back from an encountered subsurface feature can only be done to a limited extent as the pump excitation irradiance employed in PTAI is already close the damage threshold of the wafer.

### SUMMARY OF THE INVENTION

It is an object of the invention to provide for a method and a system that obviates at least one of the above mentioned drawbacks.

Additionally or alternatively, it is an object of the invention to provide a method and system for nanoscale subsurface inspection of wafers.

Additionally or alternatively, it is an object of the invention to provide a subsurface inspection method or system allowing the detection of at least small scale subsurface features and/or determining an overlay/misalignment error of the sample, such as a semiconductor wafer.

Thereto, the invention provides for a subsurface inspection method. The method can be employed for identifying internal defects or anomalies in a semiconductor wafer and/or for identifying overlay or misalignment errors in the semiconductor wafer. The method comprises: splitting a measurement laser beam into a laser probe beam and a reference laser beam; transmitting the laser probe beam to a surface of the wafer; transmitting a laser excitation pulse impinging upon a target location on the surface of the wafer to generate an ultrasound wave propagating through the wafer, wherein the ultrasound wave causes a wafer surface movement at or near the target location when reflected back from an encountered subsurface feature inside the wafer; recombining the laser probe beam and the reference laser beam in an optical interference detector; and detecting a subsurface feature inside the wafer by a deviation of a detected phase difference. At least the laser probe beam is guided through an optic prior to arriving at the optical interference detector. The optic has a dispersive characteristic dimensioned to enlarge the phase difference between the reference beam and the wave length shifted probe beam to detect a phase difference between the reflected laser probe beam and the reference beam as a result of a wavelength shift of the probe beam during or after the laser excitation pulse. Optionally, both the laser probe beam and the reference laser beam are guided through the optic.

The method can be employed for photo-thermal acoustic imaging for subsurface nano-inspection of wafers. By means of the optic, dispersion enhanced Doppler-based photo acoustic measurements or characterizations can be carried out, wherein the detection limits of the measurement is improved. Small-scale displacement of the surface of the sample can be detected as a result of the improvement. The wavelength shift or Doppler frequency shift is the result of the moving surface of the wafer and can be converted to a phase shift measured by the optical interference detector. The dispersive optic can have a wavelength dependent refractive index. This may in an advantageous way allow configuring the extent to which an altered wavelength (cf. Doppler shift) is converted into a changed phase. By using materials with a larger dispersion for the optic, and/or by increasing the path length through the optic, smaller wavelength differences can be converted to a same phase shift. Hence, in this way, the measurement sensitivity can be significantly improved. Smaller internal subsurface features, such as defects or anomalies and/or layer overlay/alignment, can be effectively detected or characterized since smaller deflections of the surface of the wafer can be detected more precisely. Additionally, internal subsurface features which are buried deeper below the surface of the wafer can be detected or characterized in an improved way. Also the scalability of the measurement method can be improved.

The photo-thermal acoustic imaging method can be also be used for three-dimensional nano-applications. Advantageously, it may no longer be necessary to increase the strength of the excitation pulse (cf. pump laser pulse) for increasing an (input) acoustic shock wave for increasing the amplitude of the wafer surface deformations resulting from the wave reflected back from an encountered subsurface feature. In this way, the risk of causing damage to the wafer being characterized can be reduced.

It is appreciated that the optic having dispersive properties can be positioned in any part of the beam path which the laser probe beam traverses after it has been reflected from the sample.

Particular changes in the phase difference between the reflected laser probe beam and the reference laser beam can indicate a presence of one or more subsurface features when a measurement is carried out on different location on the surface of the wafer. Optionally, the method steps are repeated as often as needed to generate time dependent data.

Advantageously, both the displacement (typically picometer range) and the velocity (typically meter/second range) of the surface of the wafer are taken into account, each contributing to a phase-difference between the reflected laser probe beam and the reference laser beam.

During or after the laser excitation pulse, at which the impact of the excitation pulse is (still) measurable, the phase difference between the reflected laser probe beam and the reference beam can be enhanced by means of the optic. At least the reflected laser probe beam is guided through an optic prior to arriving at an interference detector. Optionally, also the reference laser beam is guided through the optic prior to arriving at the interference detector.

Optionally, the excitation beam has a wavelength for being, at least to a good extent, absorbed by the surface material of the wafer, and the probe beam has a wavelength for being, at least to a good extent, reflected by the surface material of the wafer. Hence, the wavelength choice for at least one of the excitation beam or the probe beam may be sample specific, i.e. configured for providing good reflection/absorption for the used sample.

Optionally, the reference laser beam and the laser probe beam can be configured to arrive at different times at the sample, and, in case there is no deflection of the surface of the sample, arrive at the same time at the optical interference detector where they interfere. An irradiance on the optical interference detector depends on the phase difference between the two beams and thus contains information about the relative surface height of the surface of the wafer at two specific moments in time.

Optionally, the laser probe beam and the laser reference beam are both transmitted to the surface of the wafer. The laser reference beam is formed by a first pulse configured to arrive at the surface of the wafer synchronized with the laser excitation pulse. The laser probe beam is formed by a second pulse configured to arrive at the surface of the wafer after a predetermined delay, during which the surface of the wafer is moved as a result of the ultrasound wave reflected back from an encountered subsurface feature/structure inside the wafer.

It is appreciated that the term synchronized may imply that there exists a time relationship. It is not limited to the situation in which the events occur at the same time (cf. simultaneous). There may be a time delay before an ultrasound wave (or acoustic pulse) is obtained. The second pulse may be used for reaching the surface of the wafer after instead of during the excitation. During the laser excitation pulse material of the wafer may be in the process of warming up, so that it may be desired to enable the second pulse to arrive at the surface of the wafer in a period thereafter, when the surface is oscillating, since the moving surface can contain information or characterization data about the subsurface structure of the wafer.

Optionally, the first pulse is employed as the excitation pulse. For example, in case of a short first pulse, the surface of the wafer may not yet be moving or deflecting when the first pulse is no longer impinging on the surface.

The probe beam may be a pulsed beam or a continuous beam. For a pulsed beam, first and second pulses may be provided by a single pulse event, wherein a reference beam and a probe beam are guided through optical branches of a mutually different path length, to result in first and second pulses traveling in a same optical path. The surface shape deflecting/deforming as a result of the generated acoustic wave reflected back from a subsurface feature can be measured using two probe pulses, namely the first pulse and the second pulse. In an example, the first pulse is configured to encounter the surface of the wafer before the laser excitation pulse (i.e. pump pulse), and the second pulse is configured to encounter the surface of the wafer after the predetermined time delay after the laser excitation pulse has encountered the surface of the wafer. The distance with respect to the surface of the sample will change as a result of the deflection, resulting in a phase difference between the first pulse and the second pulse. This phase difference is detected at the optical interference detector at a certain time. A plurality of measurements at different time steps can be carried out to obtain a time-resolved measurement. Prior to reaching the optical interference detector, the reflected first pulse and second pulse are guided through the optic to enhance the measurements by enlarging the phase difference between the first pulse and the second beam. In this way, subsurface features can be detected more accurately by recording interference between the first pulse and the second pulse.

Optionally, the first and second pulses are guided through a common path. The optic is provided in the common path, wherein the second pulse is delayed relative to the first pulse at a fixed temporal delay; and wherein the optic is selected in dimension and/or refractive index to suitably measure a phase difference between the two pulses.

A pulse can be generated which is sent in two different branches, resulting in a predetermined time difference between two pulses in the two different branches. The excitation pulse may result in a (small) deflection of the surface of the wafer resulting from an acoustic wave reflected back towards said surface. The second pulse arrives during a period in which the surface is moving as a result of the photo-thermal excitation provided by the excitation pulse. The second pulse is reflected with a Doppler shifted wavelength, giving the pulse a different dispersion rate in the optic (dispersive medium). This will result in a phase difference between the first pulse and the second pulse, with respect to the situation in which the wafer surface was not deflected (i.e. neutral position). The second pulse including a Doppler shift as a result of the moving surface of the wafer will pass through the dispersive optic in a different way than the first pulse. Preferably, the time difference that is obtained between the first pulse and the second pulse are not measured because it is very small and thus impractical or difficult to accurately measure. The obtained time difference may for example be in the order of 10⁻¹⁹ seconds or even smaller. Instead, the phase difference between the first pulse and the second pulse are determined in a detector (cf. fringe formation). The difference in phase can result in a fringe pattern obtained as a result of interference in the detector, which can be determined or measured.

It is appreciated that observing the time difference between the first pulse and the second pulse can be very difficult as the two pulses overlap almost completely over time. This may result in an extremely small shift between the reference beam and the reflected probe beam. A level of overlap can be expressed as a fraction, such as a fractional shift. The fractional shift can be in the order of 10⁻⁶. For example, if a pulse lasts 100 femtoseconds, at a light velocity of 3e8 m/s the spatial dimension of a pulse is 30 micrometers. The mutual shift of the reference and probe pulse is then in the order of tens of picometers. Although it is advantageous to determine a phase difference between the first and second pulse, which is measurable by interference in a detector, it is also envisaged that a time delay or time difference is measured, if the obtained time difference is large enough for providing a desired characterization of subsurface features of the wafer.

The term common path may be linked to a type of interferometer. An optical source may be used in which a beam of light (or pulse) passes through a beam splitter, thereby obtaining two beams of light that pass through the same optical path but in opposing directions Because the optical paths of the different beam of lights have the same (total) path length, they can arrive at the detector simultaneously while illuminating the sample at different times (in case of no deflection/movement of the sample surface during sampling). However, although the two beams of light may arrive simultaneously at the detector when the surface of the wafer/sample is not moved or moving, one beam of light may impinge earlier on the surface of the sample than the other beam of light. This can be achieved by providing a predetermined optical path difference between the beams of light prior to arriving at the surface of the wafer/sample. In case the wafer/sample is moved or moving, the beam of lights will arrive slightly at different times at the detector (not simultaneous anymore), resulting in a measurable interference.

By dispersion in the optic, the second pulse can experience a different refractive index than the first pulse, and thereby the phase difference between the first and second pulse can be enhanced. The phase difference can be measured by means of interference (e.g. interferometric detector). Many possible implementations are possible for achieving this.

The first pulse and the second pulse may originate from the same (laser) source, but arrive at the surface of the wafer at a difference time because they follow different optical paths. The optical paths may be defined by for example optical arms of an interferometer, having different lengths.

Optionally, the measurement laser beam is formed by a single pulse that is split into two optical branches of different optical path lengths, and recombined in a same optical path, to result in first and second pulses of a fixed temporal delay.

A wavelength shift between the first pulse and the second pulse, induced by the dynamic movement of the wafer surface (at a certain velocity), can result in an additional phase difference when the first pulse and the second pulse are guided through the dispersive optic.

Optionally, a resulting phase difference between the reflected wavelength shifted second pulse (or laser probe beam) and the first pulse (or reference beam) obtained as a result of at least the second pulse being guided through the optic (wavelength shift obtained due to the movement of the wafer surface with a certain velocity) is greater than a resulting phase difference obtained as a result of a static deflection of the wafer surface (at or near the target location) induced by the laser excitation pulse. It is also possible that the first pulse and the second pulse are being guided through the optic.

Optionally, the resulting phase difference obtained as a result of at least the second pulse being guided through the optic is at least 2 times greater than the resulting phase difference obtained as a result of a static deflection of the wafer surface induced by the laser excitation pulse, preferably at least 5 times greater, more preferably at least 10 times greater, even more preferably at least 100 times greater.

A wavelength difference between the two pulses (or reflected laser probe beam and reference beam) can results in a particular phase difference by guiding the two pulses through the optic. This phase difference obtained at the output of the optic depends at least on one of the dispersive properties of the optic, the dimensions of the optic or the employed wavelength of the measurement laser beam. The optic can be configured such that the phase difference due to the wavelength shift (cf. Doppler) in combination with the obtained dispersion is greater than the phase difference that is already obtained due to the fact that the wafer surface is simply moved (cf. static deformation or deflection). In this way, the obtained characterization can be enhanced. Different amplification/enhancement factors can be obtained, such as 1.1, 1.5, 2, 10, 20, 25, 50, 100, 200, etc., or even greater. Preferably the amplification/enhancement is at least larger than 1.

Optionally, the optic has a thickness of at least 20 mm, more preferably at least 50 mm, even more preferably at least 100 mm. A thicker optic may increase the optical path length of the beams or pulses going therethrough, resulting in an increased phase difference. Additionally or alternatively, a higher dispersion may enable the use of an optic with a smaller thickness for achieving substantially a same result. It is appreciated that a larger thickness may at some point become detrimental as a result absorption and/or reduced transmission quality. The thickness of the optic may be chosen depending on at least one of a material of the optic, dispersion properties of the optic, wavelength of the optical beam and pulses guided therethrough, etc.

A greater dispersion provided by the optic can result in an enhanced 'separation' of the reference laser beam and the laser probe beam since the velocity of the surface of the sample deflecting is taken into account, resulting in a greater phase difference at the optical interference detector. In this way, subsurface feature extraction can be improved.

Optionally, the optic has a thickness in the range of 20-250 mm, more preferably 50-200 mm, even more preferably 100-150 mm.

The optic may be a dispersive medium. Various dispersive media can be employed for enhancing the (interferometric) sensitivity. Optionally, the optic comprises a highly dispersive medium. The optic may be made of a slow-light material.

Optionally, the optic provides a chromatic dispersion of at least 0.005 µm⁻¹, preferably at least 0.02 µm⁻¹, more preferably at least 0.1 µm⁻¹.

Optionally the optic is rod shaped, plate shaped, block shaped. Other shapes are also possible.

Optionally, the optic is made out of a transparent solid. The solid may for example be an amorphous solid, such as glass. However, other types of solids can also be employed. Many variants are possible. As an example, the following materials can be used for the optic having a described dispersion at a beam/probe wavelength of 1560nm:

| *Material* | *Dispersion @ 1560 nm* |
|---|---|
| GaP | -0.068 µm⁻¹ |
| ZnS | -0.019 µm⁻¹ |
| Si | -0.076 µm⁻¹ |
| SiO₂ | -0.012 µm⁻¹ |
| CdTe | -0.0747 µm⁻¹ |
| InP | -0.109 µm⁻¹ |
| Schott N-FS66 | -0.023 µm⁻¹ |
| Schott P-SF67 | -0.022 µm⁻¹ |

The optic can effectively increase the sensitivity. For instance, the sensitivity can be increased by more than an order of magnitude. By using materials with higher dispersion (e.g. including Si), or materials that have been specifically engineered to have extreme dispersion, the sensitivity may be improved by orders of magnitude more. This can be done without detrimental effects on the wafer, since the intensity of the pumped laser excitation pulse is not increased for creating a stronger acoustic wave such as to increase the amplitude of the resulting deflection of the surface of the wafer.

Optionally, the optic is made out of a transparent unit at least partially filled with a transparent solid, liquid, gas and/or vapor, such as an alkali atomic vapor.

Optionally, the vapor inside the glass unit is hot rubidium vapor.

Optionally, the optic comprises an antireflection coating.

Optionally, the first laser probe beam and the second laser probe beam are a multiple number of times guided through the same optic prior to arriving at the optical interference detector. In an example, by means of reflecting surfaces such as mirrors at least one of the optical beams is passed back and forth one or more times. In this way, the optical path length through the optic can be increased for at least one of the first laser probe beam and the second laser probe beam.

According to a further aspect, the invention provides for a subsurface wafer inspection system. The system can be employed for identifying internal defects or anomalies in a semiconductor wafer and/or for identifying overlay or misalignment errors in the semiconductor wafer. The system comprises a laser interferometer having a controller configured to carry out the steps of: splitting a measurement laser beam into a laser probe beam and a reference laser beam; transmitting the laser probe beam to a surface of the wafer; transmitting a laser excitation pulse impinging upon a target location on the surface of the wafer to generate an ultrasound wave propagating through the wafer, wherein the ultrasound wave causes a wafer surface movement at or near the target location when reflected back from an encountered subsurface feature inside the wafer; recombining the laser probe beam and the reference laser beam in an optical interference detector arranged for detecting a phase difference between the reflected laser probe beam and the reference beam as a result of a wavelength shift of the probe beam during or after the laser excitation pulse; and detecting a subsurface feature inside the wafer by a deviation of a detected phase difference; wherein the laser probe beam and the reference laser beam are guided through an optic prior to arriving at the optical interference detector, the optic having a dispersive characteristic dimensioned to enlarge the phase difference between the reference beam and the wave length shifted probe beam.

The optic can be arranged in any part of the beam path which the second pulse traverses after it has been reflected from the sample. The dispersive optic can be transparent and made out of one or more materials with predetermined dispersive properties tuned with an optical setup of the system. The system provides for a dispersion-enhanced Doppler-shift-based photo-thermal acoustic imaging for subsurface characterization and/or inspection of wafers or semiconductor elements, suitable for use in 3D nano-imaging.

The interferometer may be a Sagnac interferometer adapted to include said dispersive optic. Optionally, an existing interferometer is adapted and configured to include the optic. In this way, the resolution of existing interferometers can be effectively improved (e.g. a 100x better resolution), while avoiding costs involved with setting up a new system.

Optionally, the system comprises a signal processor configured to calculate the phase difference between the reference laser beam and laser probe beam reflected back from the surface of the wafer.

Optionally, a sensor scan head is used for measuring at different locations on the surface of the wafer, for example by scanning said surface. In an example, a two-dimensional sensor scan head is employed.

It is appreciated that dispersion can be regarded as a phenomenon linked to transparent materials having a wavelength-dependent refractive index. As a result of dispersion, the apparent length of a component, i.e. the optical path length as experienced by light passing through such a component, may depend on the wavelength of the light.

It will be appreciated that any of the aspects, features and options described in view of the method apply equally to the system. It will also be clear that any one or more of the above aspects, features and options can be combined.

### BRIEF DESCRIPTION OF THE DRAWING

The invention will further be elucidated on the basis of exemplary embodiments which are represented in a drawing. The exemplary embodiments are given by way of non-limitative illustration. It is noted that the figures are only schematic representations of embodiments of the invention that are given by way of non-limiting example.

In the drawing:
Fig. 1 shows a schematic diagram of an embodiment of a system;
Fig. 2 (a and b) shows a schematic diagram of an embodiment of a wafer being probed;
Fig. 3 shows a schematic diagram of an embodiment of a system; and
Fig. 4 shows a schematic diagram of a method.

### DETAILED DESCRIPTION

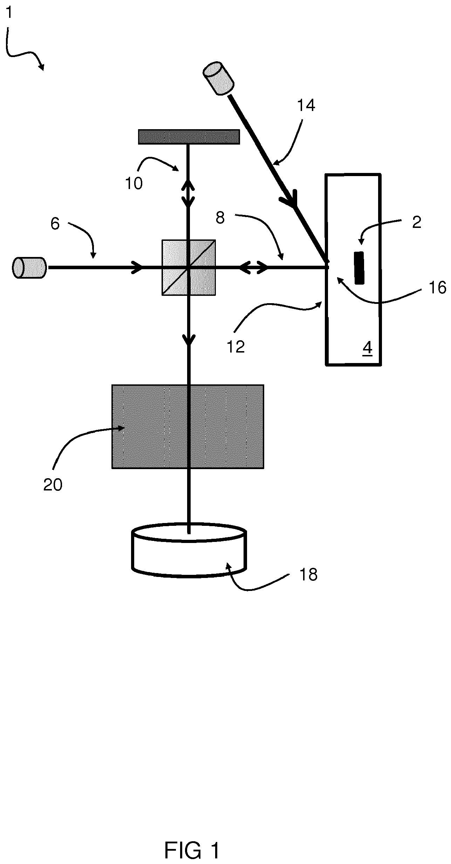

Fig. 1 shows a schematic diagram of an embodiment of a subsurface wafer inspection system 1. The method can be employed for identifying internal subsurface features, such as defects or anomalies 2 in a semiconductor wafer 4, and/or for identifying overlay or misalignment errors in the semiconductor wafer 4. The system 1 comprises a laser interferometer having a controller configured to carry out the steps of: splitting a measurement laser beam 6 into a laser probe beam 8 and a reference laser beam 10; transmitting the laser probe beam 8 to a surface 12 of the wafer 4; transmitting a laser excitation pulse 14 impinging upon a target location 16 on the surface 12 of the wafer 4 to generate an ultrasound wave propagating through the wafer 4, wherein the ultrasound wave causes a wafer surface movement at or near the target location 16 when reflected back from an encountered subsurface feature, such as defect 2 inside the wafer 4; recombining the laser probe beam 8 and the reference laser beam 10 in an optical interference detector 18 arranged for detecting a phase difference between the reflected laser probe beam 8 and the reference beam 10 as a result of a wavelength shift of the probe pulse during or after the laser excitation pulse 14; and detecting the subsurface feature 2 inside the wafer by a deviation of a detected phase difference; wherein at least the laser probe beam 8 is guided through an optic 20 prior to arriving at the optical interference detector 18, the optic 20 having a dispersive characteristic dimensioned to enlarge the phase difference between the reference beam 10 and the wave length shifted probe beam 8. In this example, both the reference laser beam 10 and the laser probe beam 8 are guided through the optic 20. However, it is also possible that only the (reflected) laser probe beam 8 is guided through the optic 20 (reference laser beam 10 not guided through the optic 20).

The system 1 enables a non-destructive wafer 4 subsurface characterization technique with a nanoscale resolution. Non-destructive testing is more feasible in terms of price, convenience and reliability. The subsurface characterization is carried out by determining the interaction of the wafer with acoustic waves, involving monitoring the effect of the acoustic wave passing through the material and reflecting as it is influenced by flaws or inhomogeneities in the wafer. Monitoring the effects can be done remotely without structural contact (using light). The laser excitation pulse 14 locally pumps sufficient energy in a short period of time causing an acoustic wave (e.g. ultrasound). The displacement of the surface 4 at or near the target location 16 of the wafer 4, which is caused by the induced acoustic wave reflected back from the internal subsurface feature 2, is determined on the basis of interference between the reflected laser probe beam 8 and reference beam 10.

Advantageously, the wavelength shift or Doppler shift is converted to an additional path-length difference between the reference beam 10 and the laser probe beam 8 by means of the dispersive optic 20 having tuned dispersive properties. In this way, the wavelength shift can be used for enhancing or amplifying the detected measurement signal at the optical interference detector 18. In an advantageous way, subsurface nano-imaging may be enabled for detection and/or characterization of nanostructures buried below the surface of the wafer 4.

In an example, the optic 20 is a block or rod of a transparent material, such as glass. Many shapes and forms can be employed. It is also envisaged that a plurality of optics are employed, for instance sequentially. A holder may be provided for holding the plurality of optics together.

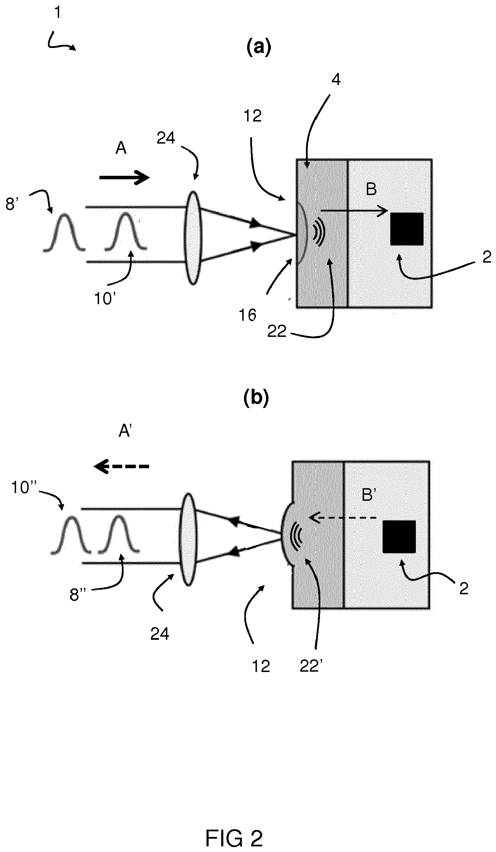

Fig. 2 shows a schematic diagram of an embodiment of a wafer 4 being probed by system 1. In this embodiment, the laser probe beam 8 and the laser reference beam 10 are both transmitted to the surface of the wafer 4 in the direction of arrow A in fig. 2(a). The laser reference beam 10 is formed by a first pulse 10' configured to arrive at the surface 12 of the wafer 4 synchronized with the laser excitation pulse 14 (not shown). The laser probe beam 8 is formed by a second pulse 8' configured to arrive at the surface 12 of the wafer 4 after a predetermined delay, during which the surface of the wafer 4 is moved as a result of the ultrasound wave reflected back from an encountered subsurface feature 2 inside the wafer 4.

The laser excitation pulse 14 irradiates the surface 12 of the wafer 4. As a result, the surface 12 is locally heated up during a relatively short period of time (e.g. femto- or picoseconds) at or near the target location 16. After this, an acoustic pressure pulse is generated resulting in an acoustic shock wave 22 travelling or propagating from near the surface 12 towards the inside (i.e. subsurface) of the wafer 4, see arrow B in fig. 2(a). When subsequently a subsurface feature 2 is encountered in an inner volume of the wafer 4, the acoustic wave 22 can at least partially be reflected back and cause movement of the surface 12 of the wafer 4 (deflection) at or near the target location 12, see arrow B' in fig. 2(b). A time resolved deflection of the surface 12 of the wafer 4 at or near the target location 12 may provide information about the internal structure of the subsurface feature 2 inside the wafer 4. Typically the movement of the surface 12 of the wafer 4 (i.e. deflection) is in the range of a few picometers occurs in the range of a few picoseconds. As a result, the instantaneous velocity of the surface 12 of the wafer 4 moving is in a meters/second range.

At the time the first pulse 10' arrives at the surface 12 of the wafer 4, the surface 12 is not moving. At the time of the second pulse 8' arrives at the surface of the wafer, the surface 12 is moving with a velocity in the order of meters per second. As a result, the second pulse 8' can have a wavelength shift. When light reflects on a (transversely) moving object, its wavelength or frequency will experience a Doppler frequency shift which can be calculated by means of the equation F'=F·((c-v)/(c+v)), wherein v is the velocity of the surface 12 of the wafer 4 reflecting the light (acting as a mirror), c is the velocity of light, F is the frequency of the source, and F' is the shifted Doppler frequency. For example, when light of 1550 nm (1.94e8 Hz) is used for probing the surface 12 of the wafer 4 moving at 1 meter/second, the resulting Doppler frequency-shift corresponds 1.3 Hz. The first pulse 10' and second pulse 8' are reflected as a reflected first pulse 10" and a reflected second pulse 8", see arrow A' in fig. 2(b). The reflected second pulse 8" will have a different frequency, and accordingly a different wavelength, than the second pulse 8' prior to arriving at the surface 12 of the sample 4. The reflected first pulse 10" will not have a wavelength shift. Advantageously, the detectability of the induced wavelength shift can be enhanced by means of the optic 20. For example, when the optic 20 is formed by a fused silica rod having a length of 10 centimeter, the optical path length difference between the nominal and the Doppler shifted beam amounts to approximately 975 picometer, which is much larger than the corresponding displacement (e.g. in picometer range) of the surface 12 of the wafer 4 as a result of the acoustic wave 22' reflected back from the encountered subsurface feature 2 within the wafer 4. In this example, an optional focusing lens 24 is arranged.

It is appreciated that a difference in path length between the first pulse 10" to the detector 18 and the second pulse 8" to the detector 18 can include a static and a dynamic contribution. The static contribution is a result of the static deformation/deflection of the surface of the sample 4, which causes the second pulse 8" to travel a smaller distance until reaching the detector 18, than the first pulse 10". The dynamic contribution is a result of the surface of the sample 4 moving with a certain velocity. Such motion of the surface of the sample 4 causes a wavelength shift (Doppler) in the reflected second pulse 8". The resulting wavelength shift in the reflected second pulse 8" results in an additional temporal distance between the first pulse 10" and the second pulse 8" as a result of the first pulse 10" and the second pulse 8" being guided through the dispersive optic 20. This additional temporal distance between the first pulse 10" and the second pulse 8" can correspond to an additional path length difference between the two pulses 10", 8", cf. the additional path length (distance) that the second pulse 8" should have traveled to result in the obtained additional temporal distance between the first pulse 10" and the second pulse 8" resulting due to the wavelength shift in the dispersive optic 20.

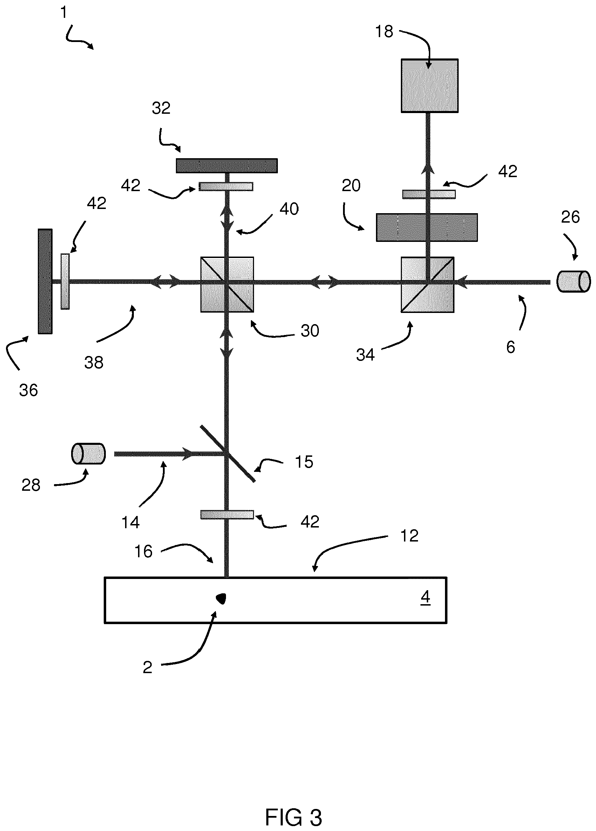

Fig. 3 shows a schematic diagram of an embodiment of a system 1. As shown in the previous embodiments, the system 1 may be configured to generate a photo-thermally generated acoustic wave (e.g. ultrasound wave) which can reflect back from an encountered subsurface feature 2, subsequently causing motion of the surface 12 of the wafer 4. The first pulse 10 and the second pulse 8 are optically guided such that after reflection they arrive at a same moment at the optical interference detector 18 in case the surface of the sample is not deformed or deflected (cf. common path length interferometer). When a deflection occurs at or near the target location 16, resulting from the photo-thermally generated acoustic wave reflected back from an encountered subsurface feature 2, the reflected first pulse 10" and the second pulse 8" will arrive at a different moment at the optical interference detector 18. Moreover, since the second pulse 8' reaches the surface of the sample in motion (deflection), the reflected second pulse 8" can have a wavelength shift with respect to the reflected first pulse 10", so that by means of the (dispersive) optic 20 the phase difference between the first pulse 10" and the wavelength shifted second pulse 8" (i.e. reflected back) can be enlarged.

At least the reflected second pulse 8" is guided through the optic 20 prior to arriving at the interference detector 18. It is possible that both the reflected first pulse 10" and the reflected second pulse 8" are guided through the optic 20 (as shown). However, it is also envisaged that only the reflected second pulse 8" is guided through the optic 20. The skilled person can make the necessary adjustments to obtain a common-path interferometric system.

In this embodiment, the first and second pulses 10, 8 are guided through a common path. The optic 20 is provided in the common path. The second pulse 8 is delayed relative to the first pulse 10 at a fixed temporal delay. The optic 20 is selected in dimension and/or refractive index to suitably measure a phase difference between the two pulses 10, 8. The measurement laser beam 6 is formed by a single pulse that is split into two optical branches of different optical path lengths, and recombined in a same optical path, to result in first and second pulses 10, 8 of a fixed temporal delay.

A first beam splitter 30 is arranged for splitting the first pulse 10' and the second pulse 8'. The first pulse 10' travels to a first mirror 32. After reflection from the first mirror 32, the first pulse 10' traverses the first beam splitter 30 and is directed onto the surface 12 of the sample 4. Optionally, a focusing lens (not shown) is provided for focusing the light on the sample. The first pulse 10' is then reflected from the surface 12 of the sample 4. The reflected first pulse 10" is then directed via the first beam splitter 30 to the second mirror 36 and then passes the first beam splitter 30 again to return to the second beam splitter 34. The reflected first pulse 10" then traverses the dispersive optic 20 prior to reaching the optical interference detector 18. The second beam splitter 34 may for example be a non-polarizing beam splitter. The first beam splitter 30 may be a polarizing beam splitter.

The second pulse 8' is first reflected off the second mirror 36 before incidence on the surface 12 of the wafer 4. The optical length of the first arm 38 between the first beam splitter 30 and the second mirror 36 is configured to be longer than the optical length of the second arm 40 between the first beam splitter 30 and the first mirror 32 by a, preferably fixed, predetermined amount. In this way, it can be ensured that the second pulse 8' arrives at the surface 12 of the wafer 4 after (i.e. time delay) the first pulse 10'. The second pulse 8' travels from the first beam splitter 30 to the second mirror 36. After reflection from the second mirror 36, the second pulse 8' traverses the first beam splitter 30 and is directed onto the surface 12 of the sample 4. The second pulse 8' is then reflected from the surface 12 of the sample 4. The reflected second pulse 8" is then directed via the first beam splitter 30 to the first mirror 32 and then passes the first beam splitter 30 again so as to return to the second beam splitter 34. The reflected second pulse 8" then also traverses the dispersive optic 20 prior to reaching the optical interference detector 18. Hence, a common path interferometric set-up can be employed. The second pulse 8" is guided to the optical interference detector 18 in which optical interference with the first pulse 10" can occur because of the common-path arrangement of the system 1. It is appreciated that the optical interference detector may comprise photodetectors. For example, variations in the optical phase difference between the first pulse 10" and the second pulse 8" reflected back from the wafer 4 can be monitored by taking the difference in output voltages of two photodetectors using a differential amplifier. Advantageously, because of the common-path arrangement, the exact temporal coincidence and interference of the reflected first pulse 10" and the reflected second pulse 8" can be guaranteed. In this example, optional waveplates 42 are arranged which are arranged for changing the polarization of light going therethrough. Such waveplates 42 may also be omitted.

Advantageously, by means of the optic 20 a nanoscale resolution can be obtained. The method can thus be used to detect subsurface features in depths of for example nanometers, or micrometers, or even deeper.

Furthermore, the common-path system 1 has no moving parts, providing an advantageous mechanical stability. The system 1 may have one or more moving optical components, for example to scan the time delay between the reference and the probe pulse onto the sample. The system 1 may be arranged to measure or image small changes in optical phase over a wide frequency range.

The system 1 may be configured to employ a train of first pulses and delayed second pulses on a target location 16 at the surface 12 of the wafer 4.

The reference beam 10 and the laser probe beam 8 originate from a same laser source 26. The laser excitation pulse 14 originates from a different laser source 28 and is guided to the surface 12 of the wafer by means of a mirror 15. However, in an example, the reference beam 10, laser probe beam 8 and the laser excitation pulse 14 may originate from a same light source.

In this example, the first pulse 10' and the second pulse 8' are incident normally on the surface of the wafer. However, it is also possible that the first pulse 10' and the second pulse 8' are at a different angle with respect to the surface of the wafer.

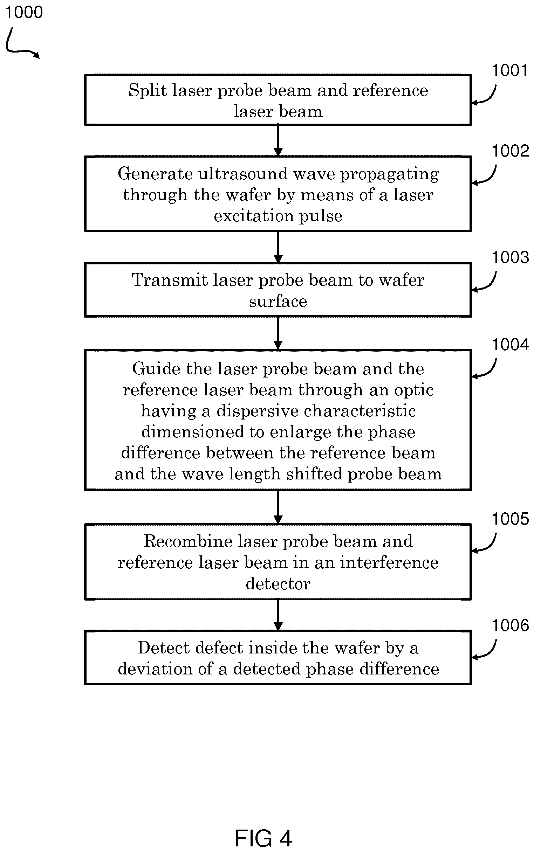

Fig. 4 shows a schematic diagram of a method 1000 for subsurface inspection method for identifying internal or subsurface features, such as for example defects or anomalies, in a semiconductor wafer and/or for determining overlay/misalignment errors in said semiconductor wafer 4. In a first step 1001, a measurement laser beam is split into a laser probe beam and a reference laser beam. In a second step 1002, a laser excitation pulse is transmitted impinging upon a target location on the surface of the wafer such as to generate an ultrasound wave which propagates through the wafer. In a third step 1003, the laser probe beam is transmitted to a surface of the wafer. It is appreciated that the third step 1003 may be performed simultaneously with or prior to the second step 1002, or vice versa. The generated ultrasound wave causes a wafer surface movement at or near the target location when reflected back from an encountered subsurface feature inside the wafer. In a fourth step 1004, at least the laser probe beam is guided through an optic having a dispersive characteristic dimensioned to enlarge the phase difference between the reference beam and the wave length shifted probe beam. Optionally, also the reference laser beam is guided through said optic. In a fifth step 1005, the laser probe beam and the reference laser beam are recombined in an optical interference detector which is arranged for detecting a phase difference between the reflected laser probe beam and the reference beam as a result of a wavelength shift of the probe beam during or after the laser excitation pulse. In a sixth step 1006, a feature inside the wafer is detected by a deviation of a detected phase difference.

When the generated ultrasound acoustic wave is reflected on a subsurface feature or structure inside the sample, the reflected acoustic wave will propagate back to the surface, and cause the surface to deform, wherein information about the three-dimensional structure of the sample can be inferred by measuring a time and position resolved wafer surface shape. By means of the optic, the precision can be improved.

The dispersive optic can be configured to transfer a difference in wavelength between the reference beam and the laser probe beam in a path-length difference therebetween. The path-length difference is added to the path-length difference resulting from a difference in the surface height of the wafer as a result of the deflection. It can be measured on the basis of interference between the reference beam and the laser probe beam.

Subsurface imaging can be carried out with increased accuracy in comparison to current subsurface measurement techniques. It may be possible to characterize subsurface features with nanometer resolution or even better, using interference measurement methods. The method can for example be utilized in semiconductor industry for inspection and/or characterization of produced wafers.

The method can improve the resolution, precision, signal-to-noise ratio, measurement time, throughput and/or detection limits of the photo-thermal acoustic imaging method.

It will be appreciated that an interferometer can be regarded as an optical system configured to split light from a single source into at least two beams that travel different optical paths, then combined again to produce interference. The resulting interference fringes in an interference detector can give information about the difference in optical path length.

It is appreciated that instead of a laser excitation beam/pulse also a different kind of optical beam/pulse can be employed, for example a LED beam pulse, a flashlamp beam/pulse, an infrared beam/pulse, etc. It is appreciated that instead of optical excitation, other forms of excitation can be employed, for example an acoustic transmitter or a mechanical device such as a piezo-type actuator.

The terms "first", "second", "third" and the like, as used herein may not denote any order, quantity, or importance, but rather are used to distinguish one element from another. They may be used to solely distinguish one entity or action from another entity or action without necessarily requiring or implying any actual such relationship or order between such entities or actions.

Herein, the invention is described with reference to specific examples of embodiments of the invention. It will, however, be evident that various modifications and changes may be made therein, without departing from the essence of the invention. For the purpose of clarity and a concise description features are described herein as part of the same or separate examples or embodiments, however, alternative embodiments having combinations of all or some of the features described in these separate embodiments are also envisaged.

The subsurface wafer inspection may be implemented in an automated wafer inspection system arranged to inspect wafers in batch or continuously.

It will be appreciated that the method may include computer implemented steps. All above mentioned steps can be computer implemented steps. Embodiments may comprise computer apparatus, wherein processes performed in computer apparatus. The invention also extends to computer programs, particularly computer programs on or in a carrier, adapted for putting the invention into practice. The program may be in the form of source or object code or in any other form suitable for use in the implementation of the processes according to the invention. The carrier may be any entity or device capable of carrying the program. For example, the carrier may comprise a storage medium, such as a ROM, for example a semiconductor ROM or hard disk. Further, the carrier may be a transmissible carrier such as an electrical or optical signal which may be conveyed via electrical or optical cable or by radio or other means, e.g. via the internet or cloud.

Some embodiments may be implemented, for example, using a machine or tangible computer-readable medium or article which may store an instruction or a set of instructions that, if executed by a machine, may cause the machine to perform a method and/or operations in accordance with the embodiments.

Various embodiments may be implemented using hardware elements, software elements, or a combination of both. Examples of hardware elements may include processors, microprocessors, circuits, application specific integrated circuits (ASIC), programmable logic devices (PLD), digital signal processors (DSP), field programmable gate array (FPGA), logic gates, registers, semiconductor device, microchips, chip sets, et cetera. Examples of software may include software components, programs, applications, computer programs, application programs, system programs, machine programs, operating system software, mobile apps, middleware, firmware, software modules, routines, subroutines, functions, computer implemented methods, procedures, software interfaces, application program interfaces (API), methods, instruction sets, computing code, computer code, et cetera.

The sequence of operations (or steps) is not limited to the order presented in the figures and/or claims unless specifically indicated otherwise. Furthermore, the order in which various described method steps are performed may be changed in alternative embodiments, and in other alternative embodiments one or more method steps may be skipped altogether.

Herein, the invention is described with reference to specific examples of embodiments of the invention. It will, however, be evident that various modifications, variations, alternatives and changes may be made therein, without departing from the essence of the invention. For the purpose of clarity and a concise description features are described herein as part of the same or separate embodiments, however, alternative embodiments having combinations of all or some of the features described in these separate embodiments are also envisaged and understood to fall within the framework of the invention as outlined by the claims. The specifications, figures and examples are, accordingly, to be regarded in an illustrative sense rather than in a restrictive sense. The invention is intended to embrace all alternatives, modifications and variations which fall within the spirit and scope of the appended claims. Further, many of the elements that are described are functional entities that may be implemented as discrete or distributed components or in conjunction with other components, in any suitable combination and location.

In the claims, any reference signs placed between parentheses shall not be construed as limiting the claim. The word 'comprising' does not exclude the presence of other features or steps than those listed in a claim. Furthermore, the words 'a' and 'an' shall not be construed as limited to 'only one', but instead are used to mean 'at least one', and do not exclude a plurality. The mere fact that certain measures are recited in mutually different claims does not indicate that a combination of these measures cannot be used to an advantage.

## Claims

1. A subsurface inspection method comprising the steps of:
splitting a measurement laser beam into a laser probe beam and a reference laser beam,
transmitting the laser probe beam to a surface of the wafer, transmitting a laser excitation pulse impinging upon a target location on the surface of the wafer to generate an ultrasound wave propagating through the wafer, wherein the ultrasound wave causes a wafer surface movement at or near the target location when reflected back from an encountered subsurface feature inside the wafer,
recombining the laser probe beam and the reference laser beam in an optical interference detector, and
detecting the subsurface feature inside the wafer by a deviation of a detected phase difference,
wherein at least the laser probe beam is guided through an optic prior to arriving at the optical interference detector, the optic having a dispersive characteristic dimensioned to enlarge the phase difference between the reference beam and the wave length shifted probe beam to detect a phase difference between the reflected laser probe beam and the reference beam as a result of a wavelength shift of the probe beam during or after the laser excitation pulse.

2. Subsurface inspection method according to claim 1, wherein the laser probe beam and the laser reference beam are both transmitted to the surface of the wafer, the laser reference beam formed by a first pulse configured to arrive at the surface of the wafer synchronized with the laser excitation pulse, and the laser probe beam formed by a second pulse configured to arrive at the surface of the wafer after a predetermined delay, during which the surface of the wafer is moved as a result of the ultrasound wave reflected back from an encountered subsurface defect inside the wafer.

3. Subsurface inspection method according to claim 2, wherein the first and second pulses are guided through a common path, said optic being provided in the common path, wherein the second pulse is delayed relative to the first pulse at a fixed temporal delay; and wherein the optic is selected in dimension and/or refractive index to suitably measure a phase difference between the two pulses.

4. Subsurface inspection method according to claim 2 or 3, wherein the measurement laser beam is formed by a single pulse that is split into two optical branches of different optical path lengths, and recombined in a same optical path, to result in first and second pulses of a fixed temporal delay.

5. Subsurface inspection method according to any one of the preceding claims, wherein a resulting phase difference between the reflected wavelength shifted second pulse and the first pulse obtained as a result of at least the second pulse being guided through the optic is greater than a resulting phase difference obtained as a result of a static deflection of the wafer surface induced by the laser excitation pulse.

6. Subsurface inspection method according to any one of the preceding claims, wherein the resulting phase difference obtained as a result of at least the second pulse being guided through the optic is at least 2 times greater than the resulting phase difference obtained as a result of a static deflection of the wafer surface induced by the laser excitation pulse, preferably at least 5 times greater, more preferably at least 10 times greater, even more preferably at least 100 times greater.

7. Subsurface inspection method according to any one of the preceding claims, wherein the optic has a thickness of at least 20 mm, more preferably at least 50 mm, even more preferably at least 100 mm.

8. Subsurface inspection method according to any one of the preceding claims, wherein the optic has a thickness in the range of 20-250 mm, more preferably 50-200 mm, even more preferably 100-150 mm.

9. Subsurface inspection method according to any one of the preceding claims, wherein the optic provides a chromatic dispersion of at least 0.005 µm⁻¹, preferably at least 0.02 µm⁻¹, more preferably at least 0.1 µm⁻¹.

10. Subsurface inspection method according to any one of the preceding claims, wherein the optic is made out of a transparent solid or wherein the optic is made out of a transparent glass unit at least partially filled with a transparent solid, liquid, gas and/or vapor.

11. Subsurface inspection method according to any one of the preceding claims, wherein the first laser probe beam and the second laser probe beam are a multiple number of times guided through the same optic prior to arriving at the optical interference detector.

12. A subsurface wafer inspection system comprising a laser interferometer having a controller configured to carry out the steps of:
splitting a measurement laser beam into a laser probe beam and a reference laser beam,
transmitting the laser probe beam to a surface of the wafer, transmitting a laser excitation pulse impinging upon a target location on the surface of the wafer to generate an ultrasound wave propagating through the wafer, wherein the ultrasound wave causes a wafer surface movement at or near the target location when reflected back from an encountered subsurface feature inside the wafer,
recombining the laser probe beam and the reference laser beam in an optical interference detector, and
detecting the subsurface feature inside the wafer by a deviation of a detected phase difference,
wherein at least the laser probe beam is guided through an optic prior to arriving at the optical interference detector, the optic having a dispersive characteristic dimensioned to enlarge the phase difference between the reference beam and the wave length shifted probe beam to detect a phase difference between the reflected laser probe beam and the reference beam as a result of a wavelength shift of the probe beam during or after the laser excitation pulse.
